# EUROPÄISCHE PATENTANMELDUNG

(11) **EP 1 103 272 A2**
(43) Veröffentlichungstag der Anmeldung: **30.05.2001**
(21) Anmeldenummer: 00126088.4
(22) Anmeldetag: 29.11.2000
(51) Int. Cl.: A61K 38/48, A61P 17/00, A61P 19/00, A61P 9/10, A61P 35/00, A61P 37/00, A61P 13/12, A61P 39/02

(54) **Beeinflussung von TGF-Beta durch proteolytische Enzyme**

(30) Priorität: 29.11.1999 DE 19957318
(71) Anmelder: MUCOS Pharma GmbH & Co., D-82538 Geretsried (DE)
(72) Erfinder: Ransberger, Karl, 82402 Seeshaupt (DE); Stauder, Gerhard, 82538 Geretsried (DE); Desser, Lucia, 1130 Wien (AT)
(74) Vertreter: Grünecker, Kinkeldey, Stockmair & Schwanhäusser Anwaltssozietät

(57) **Zusammenfassung**

Die vorliegende Erfindung betrifft die Verwendung von mindestens einem proteolytischen Enzym zur Beeinflussung von TGF-β. Bevorzugte proteolytische Enzyme sind Trypsin, Chymotrypsin, Bromelain und Papain. Zusätzlich kann Rutosid verwendet werden. Erfindungsgemäß können proteolytische Enzyme sowie Kombinationspräparate dieser Enzyme mit Rutosid zur Behandlung verschiedener Erkrankungen und pathologischer Zustände verwendet werden, bei denen ein erhöhter TGF-β-Spiegel von Bedeutung ist, wie zum Beispiel Fibrosen, Arteriosklerosen, Verbrennungen, chronische Infektionen, insbesondere rheumatoide Arthritis, Tumoren, Abstoßungsreaktionen gegen Implantate oder Transplantate und Diabetes-assoziierte Erkrankungen wie diabetogene Nephropathie sowie zur Vorbeugung der Nebenwirkungen einer Bleomycin-Chemotherapie.

## Beschreibung

Die vorliegende Erfindung betrifft die Verwendung proteolytischer Enzyme zur Beeinflussung von TGF-β und ein Verfahren zur Beeinflussung von TGF-β durch proteolytische Enzyme.

TGF-β (Transforming Growth Factor Beta) ist ein multifunktioneller Wachstumsfaktor, der an Regulationsprozessen im Immunsystem beteiligt ist. TGF-β ist ein homodimeres Peptid mit einem Molekulargewicht von 25 kD und mindestens drei Isoformen (TGF-β 1-3), die eine 70-80%ige Sequenzhomologie zueinander aufweisen. Inwieweit diese drei Isoformen unterschiedliche Eigenschaften haben, ist noch weitgehend unbekannt.

TGF-β wird in den Megakaryozyten als biologisch inaktives Molekül synthetisiert und in den α-Granulae der Thrombozyten gespeichert. Außerdem wird der Wachstumsfaktor von aktivierten T- und B-Zellen, ebenso Monozyten, Chondrozyten, Fibroblasten, Keratinozyten, Astrozyten und Tumorzellen synthetisiert. Die bei den Wundheilungsprozessen auftretenden hohen Mengen an TGF-β stammen wahrscheinlich aus den Thrombozyten.

Die biologisch inaktive Form von TGF-β hat eine Halbwertzeit von etwa 2 Stunden, die aktive von nur wenigen Minuten (Giodice, G. del und Crow, A. K. *Lupus 2:* 213-220, 1993).

In der Literatur wurde beschrieben, daß TGF-β die Synthese von IL-1, IL-6, IFN-γ, TNF-α, TNF-β, IL-2 und IL-3 und die Expression von IL-2-Rezeptoren hemmt (Derynck, R. *The Cytokine Handbook,* 2. Ausgabe, Angus Thomson, Acad. Press S. 319-343, 1994) und die Synthese von Prostaglandin-E2 stimuliert (McAnulty et al., Hernandez Rodriguez, N.A., et al. *Biochem. J*. 321, S. 639-643, 1997). Da TGF-β ebenfalls die Bildung von extrazellulärer Matrix und damit die Narbenbildung anregt (Mutsaers, S.E., Bishop, J.E., McGrouther, G., Laurent, G.J., *Int J. Biochem. Cell Biol. 29, S. 5-17,* 1997), ist er ein wichtiger Faktor bei der Wundheilung. Die Expression von Integrinen (Adhäsionsmoleküle, die der Verbindung zwischen Zelle und extrazellulärer Matrix dienen) wird ebenfalls von TGF-ß stimuliert (Basson, C.T., Kocher, O., Basson, M.D. Asis, A., Madri, J.A., *J*. *Cell Physiol.* 153, S. 118-128, 1992).

TGF-β ist ein positiv autokriner Wachstumsfaktor: er stimuliert seine eigene Synthese (Wahl, S. M., *J*. *Clin. Immunol.* 12, S. 61-73, 1992). Diese positive Rückkoppelung ist jedoch auch die Ursache dafür, daß es unter verschiedenen Bedingungen zu einer unkontrollierten Überproduktion von TGF-β und dadurch zu für den Organismus schwerwiegenden Veränderungen kommt.

Die unkontrollierte Überproduktion von TGF-β führt zu einer Vermehrung des Bindegewebes im jeweiligen Organ (Fibrosen) und zu einer allgemeinen Niedermodulierung des Immunsystems, die u.a. zur Schwächung des Abwehrsystems gegen Tumore führt. Ausgelöst wird diese u.a. durch Hemmfaktoren (z.B. Decorin) nicht mehr kontrollierbare Überproduktion von TGF-β z.B. durch radioaktive Bestrahlung, durch Medikamente wie Bleomycin, durch erhöhten Glucose-Gehalt im Blut, durch chronischen Alkoholkonsum oder durch Viren (Hepatitis C).

Aufgabe der vorliegenden Erfindung ist es, eine neue Möglichkeit zur therapeutischen Behandlung von Erkrankungen bereitzustellen, die auf die unkontrollierte Überproduktion von TGF-β zurückzuführen sind.

Erfindungsgemäß wird diese Aufgabe durch die Verwendung von mindestens einem proteolytischen Enzym zur Beeinflussung von TGF-β gelöst.

Es wurde überraschenderweise gefunden, daß die Verwendung proteolytischer Enzyme zur Behandlung von Erkrankungen, bei denen eine Überproduktion von TGF-β festgestellt wurde, zu einer Reduzierung der Werte dieses Wachstumsfaktors führt, die mit einer Besserung des Zustandes des Patienten einhergeht.

In einer bevorzugten Ausführungsform der Erfindung wird als proteolytisches Enzym Trypsin, Chymotrypsin, Bromelain oder Papain oder eine Kombination von mehreren dieser Enzyme verwendet.

Die erfindungsgemäß verwendeten Enzyme lassen sich kostengünstig aus den folgenden Rohmaterialien isolieren.

Bromelain ist ein proteolytisch wirksames Enzym, das aus dem Preßsaft der Ananas *(Ananas comosus)* isoliert wird. Das Molekulargewicht des Glykoproteins Bromelain beträgt 25 kD und das Molekül enthält einen Oligosaccharidteil aus drei Mol Mannose, aus je ein Mol D-Xylose, L-Fucose und aus zwei Mol N-Acetyl-D-Glucosamin sowie eine reaktive Sulfhydrylgruppe. Dieses Heptasaccharid ist kovalent an Asparagin des Peptid-Teils gebunden. Die für pharmazeutische Zwecke genutzte, gereinigte Bromelain-Präparation ist ein farbloses bis gelblich-braunes, amorphes Pulver, das sich gut in Wasser löst. In Laborversuchen wurde gezeigt, daß Bromelain antiphlogistische Wirkung hat. Außerdem bewirkt das Enzym eine Verlängerung der Blutungs- und Prothrombin-Zeit, eine Hemmung der Thrombozyten-Aggregation und eine Erhöhung von Antibiotika-Serumspiegel. In der Klinik wird Bromelain zur Förderung der Wundheilung bei akuten posttraumatischen oder postoperativen Schwellungszuständen und zur Enzymsubstitution bei Verdauungsbeschwerden im Rahmen von Pankreas-Erkrankungen mit unzureichender Fermentproduktion innerlich angewendet.

Papain ist ein proteolytisches Enzym, das aus dem Milchsaft der unreifen, fleischigen Früchte des Melonenbaums *Carica papaya* gewonnen wird. Reines Papain ist ein kristallines Polypeptid mit einem Molekulargewicht von 23.350 Dalton, das aus einer Kette von 212 Aminosäureresten mit 4 Disulfid-Brücken besteht; Sequenz und Raumstruktur sind bekannt. In der Klinik dient Papain bereits zur Unterstützung der enzymatischen Verdauung, zur enzymatischen Wundreinigung und als Zusatz zu Zahnprothese-Reinigungsmitteln. Für Spezialzwecke werden Papain-Präparate an Kunststoffpolymere oder an Agaroseträger gebunden angeboten. Papain ist auch als Katalysator zur Synthese von Oligopeptiden verwendet worden.

Trypsin ist ein im Pankreas gebildetes proteolytisches Enzym, das in Verbindung mit anderen Enzymen bereits therapeutisch eingesetzt wird. Es gehört zu den Serin-Proteasen. Kristallines Trypsin hat ein Molekulargewicht von ca. 23.300 Dalton, ist in Wasser, nicht aber in Alkohol löslich, besitzt ein Wirkungsoptimum bei pH 7-9 und spaltet Peptid-Ketten spezifisch carboxyseitig der basischen Aminosäurereste L-Lysin und L-Arginin. Die räumliche Struktur des aus 223 Aminosäuren bestehenden Trypsins ist bekannt.

Die Bezeichnung Chymotrypsin umfaßt eine Familie von proteinspaltenden Verdauungsenzymen des Dünndarms der Wirbeltiere, die in Form ihrer inaktiven Vorstufe Chymotrypsinogen in der Bauchspeicheldrüse gebildet werden. Die Aktivierung der Chymotrypsine aus Chymotrypsinogenen erfolgt autokatalytisch durch Spuren von Chemotrypsin und wird durch bestimmte Inhibitoren (z.B. Aprotinin) reguliert. Das α-Chymotrypsin, das aus drei Polypeptid-Ketten mit insgesamt 245 Aminosäuren-Resten besteht und ein Molekulargewicht von ca. 25 kD aufweist, hydrolysiert Proteine, Peptide, Aminosäureester und Amide spezifisch an der Carboxy-Gruppe hydrophober Aminosäuren (Phenylalanin, Tryptophan und Tyrosin). Daher ist Chymotrypsin ein wichtiges Hilfsmittel bei der Sequenzierung von Proteinen. In der Klinik wird Chymotrypsin zur Behandlung von Hämatomen, Ödemen und Entzündungen angewendet.

Eine besonders gute Wirksamkeit zeigt sich bei der erfindungsgemäßen Verwendung einer Kombination der Enzyme Bromelain, Papain, Chymotrypsin und/oder Trypsin. Neben der bemerkenswerten und unerwarteten Wirkung dieser Enzyme hat die kombinierte Verwendung der genannten Enzyme den Vorteil, daß auch bei einer Langzeitanwendung keine schädigenden Nebenwirkungen auftreten.

In einer bevorzugten Ausführungsform enthält das Enzympräparat 20 bis 100 mg Bromelain, 40 bis 120 mg Papain, 10 bis 50 mg Chymotrypsin und 10 bis 50 mg Trypsin.

Besonders bevorzugt enthält das Enzympräparat zur Beeinflussung von TGF-β 40 mg Trypsin, 40 mg Chymotrypsin und 100 mg Papain.

In einer weiteren Ausführungsform der Erfindung enthält das zur Beeinflussung von TGF-β verwendete Enzympräparat neben einem oder mehreren proteolytischen Enzymen auch Rutosid. Rutosid ist ein Glycosid, das zu den Flavonoiden gehört.

Zur Beeinflussung von TGF-β wird besonders bevorzugt eine Kombination von 45 mg Bromelain, 60 mg Papain, 1 mg Chymotrypsin, 24 mg Trypsin und 50 mg Rutosid x 3H₂O verwendet.

In einer anderen besonders bevorzugten Ausführungsform wird eine Kombination von 48 mg Trypsin, 90 mg Bromelain und 100 mg Rutosid x 3H₂O verwendet.

Die Wirkung der erfindungsgemäßen proteolytischen Enzyme wird durch alpha-2-Makroglobulin (α2M) vermittelt. Nach der Resorption im Organismus werden die Enzyme an dieses Serumprotein gebunden. α2M ist ein homotetrameres Plasma-Glykoprotein (718 KD), das Proteinasen bindet. Dieser α2M-Proteinase-Komplex wird z.B. an Rezeptoren der Makrophogen, Hepatozyten oder Fibroblasten gebunden. Man unterscheidet zwischen der "slow"-Form des α2M ohne Proteinasen und der "fast"-Form des α2M-Proteinase-Komplexes. Beide Formen können TGF-β binden: Während die "slow"-Form als Träger von TGF-β dient, der seine Aktivität behält und daher an Rezeptoren der Zellen binden und seine Funktion erfüllen kann, wird TGF-β von der "fast"-Form irreversibel gebunden und dadurch inaktiviert. Hohe Konzentrationen der"fast"-Form von α2M sind imstande, hohe Konzentrationen von TGFβ zu binden und zu inaktivieren.

Unkontrollierte Überproduktion von TGF-β, wie sie bei einer Reihe von pathologischen Zuständen beschrieben wurde, kann durch hohe Konzentrationen des Komplexes α2M-Proteinase ("fast"-Form) reduziert werden. Werden proteolytische Enzyme oral verabreicht, so können sie resorbiert und im Blut an das α2M gebunden werden. Dadurch wechselt das α2M von der "slow"-Form in die "fast"-Form und bindet irreversibel TGF-β. Die "fast"-Form wird dann rasch phagozytiert. Dem Organismus verabreichte proteolytische Enzyme vermehren die "fast"-Form des α2M, was zu einer vermehrten Inaktivierung von TGF-β führt. Bei Verringerung der TGF-β Konzentration wird seine Neusynthese ebenfalls vermindert, da TGF-β ein positiv autokriner Wachstumsfaktor ist.

Das Präparat kann weiterhin alle üblichen Hilfs- und/oder Trägerstoffe enthalten. Als Hilfs- und Trägerstoffe kommen z.B. Laktose, Magnesiumstearat, Stearinsäure, Talkum, Methacrylsäure, Copolymerisat Typ A, Shellack, Makrogol 6000, Dibutylphthalat, Vanillin, Titandioxid, weißer Ton, Polyindon, gelbes Wachs und Carnaubawachs in Frage.

Erfindungsgemäß können proteolytische Enzyme einzeln, als Kombination oder zusammen mit Rutosid zur Beeinflussung von TGF-β bei der Behandlung von Fibrosen verwendet werden. In der Klinik werden Fibrosen als der Endzustand eines chronisch entzündlichen Prozesses mit Vermehrung des Bindegewebes definiert. Bei Patienten mit Lungen-, Leber- oder Nierenfibrose hat man eine TGF-β-Überproduktion festgestellt, die mit der Vermehrung des Bindegewebes und der Narbenbildung in Zusammenhang steht. Bei der Leberfibrose, die bei pathologischen Zuständen wie Leberzirrhose oder Stauungsleber auftritt, werden in einem ersten Stadium der Krankheit zugrundegegangene Leberzellen durch kollagenes Bindegewebe ersetzt. Bei der Lungenfibrose wird das Lungengerüst narbig umgebaut. Die Verwendung von proteolytischen Enzymen bewirkt eine Reduzierung der TGF-β-Werte bei Patienten im Anfangsstadium der Krankheit und verhindert dadurch die Fibrosierungsprozesse. Auch bei Osteomyelofibrosen, das Endstadium vieler hämatologischer Erkrankungen, wurde ein hoher TGF-β-Gehalt beobachtet. Durch Anwendung der erfindunsgemäßen Enzymtherapie kann der hohe TGF-β-Gehalt gesenkt werden und dadurch der Prozeß der Krankheit wesentlich verlangsamt werden.

Eine weitere Verwendung der erfindungsgemäßen Präparate betrifft die Vorbeugung der Nebenwirkungen einer Behandlung mit dem Chemotherapeutikum Bleomycin. Kahlil, N., O'Connor, R.N., Flanders, K.C. und Unruh, H. *(Am. J. Resp. Cell Mol. Biol.* 14, S. 131-138, 1996) beschreiben, daß eine Behandlung mit Bleomycin zu einer Überproduktion von TGF-β und dadurch zur Entwicklung von Lungenfibrosen führt. Diese Nebenwirkungen werden durch Verwendung der erfindungsgemäßen Präparate vorgebeugt. Ferner werden proteolytische Enzyme erfindungsgemäß bei der Behandlung von chronischen Entzündungszuständen, beispielsweise rheumatoider Arthritis, verwendet. Auch hier hat man einen erhöhten Gehalt an TGF-β im Serum beobachtet (Klareskog, L., Ronnelid, J., Holm, G.J., *J*. *Intern. Med.* 238:191-206, 1995). Durch die Verwendung von proteolytischen Enzymen einzeln, als Kombination oder zusammen mit Rutosid wird die Konzentration von TGF-β im Serum dieser Patienten reduziert.

Arteriosklerose ist eine der wichtigsten Herz- und Kreislauferkrankungen und wird durch eine krankhafte Veränderung der Arterien mit Verhärtung, Verdickung, Elastizitätsverlust und Lichtungseinengung der Blutgefäße verursacht. Die Veränderung der Gefäßwand kann u.a. durch die Proliferation von Bindegewebs- und Muskelzellen mit gesteigerter Kollagen- und Elastinsynthese hervorgerufen werden. Auch hier ist eine unkontrollierte Überproduktion von TGF-β an der Entstehung dieses pathologischen Zustandes beteiligt. Gemäß der Erfindung werden zur Beeinflussung von TGF-β bei der Behandlung von Arteriosklerose proteolytische Enzyme einzeln, als Kombination oder zusammen mit Rutosid verwendet.

Weitere erfindungsgemäße Verwendungen proteolytischer Enzyme schließen die Behandlung von Verbrennungen und die Vorbeugung von Abstoßungsreaktionen gegen Implantate und Transplantate ein, bei denen eine unkontrollierte Überproduktion von TGF-β beobachtet wurde. Bei der Behandlung von Tumoren und insbesondere bei der Unterdrückung der Metastasierung und der Reduzierung der Immunsuppression hat sich die Verwendung proteolytischer Enzyme als vorteilhaft erwiesen.

Erfindungsgemäß können proteolytische Enzyme auch bei der Behandlung von Patienten verwendet werden, die einer radioaktiven Bestrahlung unterzogen wurden. Bei solchen Patienten beobachtet man oft eine Vermehrung des Bindegewebes insbesondere in der Lunge (Strahlenfibrose), die mit erhöhten Konzentrationen von TGF-β im Serum einhergeht. Die orale Verabreichung proteolytischer Enzyme führt zu einer Reduzierung der TGF-β-Konzentration im bestrahlten Gewebe und dadurch zu einer Verbesserung des Krankheitsbildes.

Weiterhin können proteolytische Enzyme erfindungsgemäß auch bei der Behandlung von Diabetes-assoziierten Erkrankungen verwendet werden. In einer bevorzugten Ausführungsform ist die mit Diabetes-assoziierte Erkrankung die diabetogene Nephropathie. Die Entwicklung der diabetogenen Nephropathie (d.h. diabetogene Nierenkrankheit) ist eine der häufigsten und ernsthaftesten Komplikationen von Diabetes und tritt trotz Insulin-Behandlung in annähernd der Hälfte der Patienten auf.

Die Entstehung von Nephrophathien bei Diabetes-Patienten wird mit der durch erhöhte Glucosewerte im Blut verursachten Überproduktion von TGF-β in Verbindung gebracht (Kolm-Litty, V., Sauer, U., Nerlich, A., Lehmann, R., Schleicher, eds., *J*. *of Clin Invest.* 101, S. 160-169, 1998; Rieser, B. L., Cortes, P., Yee, J., Sharba, A.K., Asano, K., et al. *J. of the American Soc. of the Nephrology 9,* S.827-836,1998). Das dominante histologische Merkmal der diabetogenen Nephropathie ist die Expansion der extrazellulären Matrix in den Mesangial-Bereichen der Glomeruli mit nachfolgendem Kapilarverschluß und Sklerose. Die Ausdehnung der Mesangial-Matrix zeigt eine starke Korrelation mit dem Beginn der klinischen Symptome der Proteinurie, Bluthochdruck und Nierenversagen.

Matrixabnormalitäten bei Glomerulonephritis und diabetogener Nephropathie, in denen die Ansammlung der extrazellulären Matrix pathologisch wird, zeigen deutliche Ähnlichkeiten, welches auf die Beteiligung von TGF-β bei diabetogener Nephropathie hindeutet. Studien zeigten, daß Glomeruli diabetogener Ratten eine deutliche Erhöhung der Expression von TGF-β1 mRNA und TGF-β1 Protein aufwiesen. Die Glomeruli zeigten weiterhin eine erhöhte Ablagerung einer alternativ gespleißten Form der extrazellulären Matrix-Proteine, Fibronektin und Tenascin. In Bezug auf letztere Komponenten ist bekannt, daß diese durch TGF-β induziert werden. Somit ergibt sich, daß TGF-β auch zur Entwicklung von diabetogener Nephropathie beiträgt.

Durch die Verwendung der erfindungsgemäßen Präparate kann eine übermäßige Produktion von TGF-β verhindert oder vermindert werden, und so die Entstehung bzw. die Progression einer Diabetes-assoziierten Erkrankung, insbesondere einer diabetogenen Nephropathie bei Diabetes-Patienten verhindert werden.

Die Erfindung betrifft weiterhin ein Verfahren zur Beeinflussung von TGF-β, wobei TGF-β mit einem oder mehreren proteolytischen Enzymen in Kontakt gebracht wird. Vorzugsweise wird zusätzlich Rutosid verwendet.

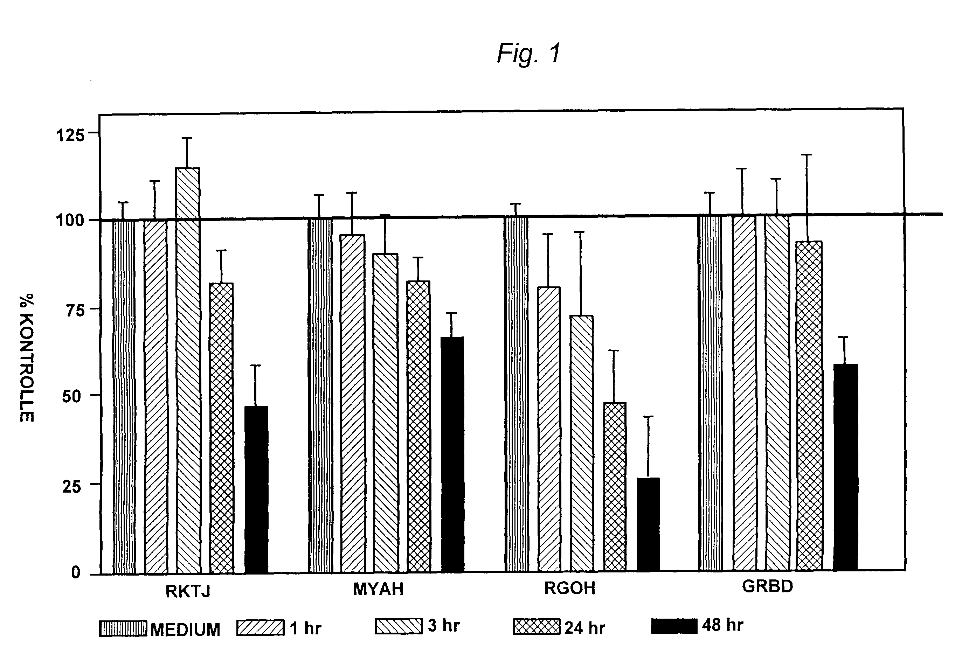

Figur 1 zeigt die Ergebnisse des in Beispiel 1 beschriebenen Versuchs in Form eines Balkendiagramms.

Die folgenden Beispiele sollen die Erfindung im einzelnen erläutern.

### AUSFÜHRUNGSBEISPIEL 1

Die Expression des Vitronektinrezeptors (avβ3) auf Melanomzellen korreliert mit deren Fähigkeit Metastasen zu bilden (Cheresh et al., *Cell* 57; S. 59-65, 1989). Es ist bekannt, daß TGF-β die Expression von Vitronektirezeptoren in Tumorzellen stimuliert (Koli, K., Lohi, J. Hautanen, A. und Keski-Oja, *J*. *Eur. J. Biochem.* 199, S. 337-345, 1991).

Melanomzellen wurden *in vitro* mit einer Kombination aus Papain, Trypsin und Chymotrypsin (0,5 x10⁻⁶ mol/l) in RPMI-Medium mit 10% FKS inkubiert und die Expression des Vitronektinrezeptors zwischen 1 und 48 Stunden nach Inkubation bestimmt. Wie aus Figur 1 ersichtlich, nimmt die Expression des Vitronektinrezeptors in den behandelten Melanomzellen abhängig von der Zeit ab.

Melanomzellen produzieren TGF-β. Mit Hilfe der RT/PCR-Methode wurde die TGF-β-mRNA in Melanomzellen, die mit Medium oder mit proteolytischen Enzymen (Kombination aus Papain, Trypsin und Chymotrypsin) inkubiert worden waren, bestimmt. Die Ergebnisse werden in Tabelle 1 gezeigt. Dabei war der Inkubationsansatz wie folgt:

Menschliche Melanomzellen (RKTJ, MYAH, SKMel, SHTJ) wurden in einer Konzentration von 1 x 10⁶ Zellen/ml Medium (RPMI + 10% FKS) in Kulturflaschen gezüchtet. Das Enzympräprat (proteolytisches Enzympräparat bestehend aus Papain, Trypsin und Chymotrypsin) wurde in einer Konzentration von (0,5 x 10⁻⁶ mol/l) zugesetzt. Nach 24 Stunden wurden die Zellen geerntet und die mRNA für TGF-β1 mit der RT/PCR-Methode nach Lupetti, R., Mortarini, R., Panceri, P., Sensi, M., Anichini, A. *Int. J. Cancer* 66, S. 1 10-116, 1996 bestimmt.

**TABELLE 1**

| mRNA-Expression (RT/PCR) von TGF-β1 in Melanomzellen, inkubiert mit Medium oder proteolytischen Enzymen | | |
|---|---|---|
| **menschliche Melanome** | **Medium (RPMI+10% FKS)** | **Medium + Proteolytische Enzyme** |
| RKTJ | 1,91 | 0,86 |
| MYAH | 0,54 | 0,41 |
| SKMEL | 0,75 | 0,41 |
| SHTJ | 0,34 | 0,13 |
| RALL | 0,64 | 0,57 |

Die Werte sind in optischer Dichte angegeben.

### AUSFÜHRUNGSBEISPIEL 2

Patienten mit rheumatischer Arthritis haben einen erhöhten Gehalt an TGF-β im Serum (Klareskog et al., *J*. *Intern. Med.* 238:191-206, 1995). Wie die Ergebnisse in Tabelle 2 zeigen, wird die Konzentration von TGF-β im Serum von Patienten mit rheumatischer Arthritis durch die Behandlung mit einer Kombination aus Bromelain, Trypsin und Rutosid (3x3 Tabletten täglich; jede Tablette enthält 90 mg Bromelain, 48 mg Trypsin und 100 mg Rutosid x 3H₂O) reduziert.

**TABELLE 2**

| TGF-β (β1-3) (pg/ml) im Serum von Patienten mit rheumatischer Arthritis: Enzymtherapie (Kombination aus Bromelain, Trypsin und Rutosid) | | | | | | |
|---|---|---|---|---|---|---|
| | **Start** | **1. Monat** | **2. Monat** | **3. Monat** | **4. Monat** | **5. Monat** |
| 1. Patient | 3563 | 3085 | 2458 | 2288 | 2271 | |
| 2. Patient | 2501 | 1981 | 1679 | 1945 | | |
| 3. Patient | 3102 | 2907 | 2830 | 2742 | | 2124 |
| 4. Patient | 3520 | 3007 | 2812 | | 2284 | 2225 |

## Patentansprüche

1. Verwendung von mindestens einem proteolytischen Enzym zur Beeinflussung von TGF-β.

2. Verwendung nach Anspruch 1, **dadurch gekennzeichnet**, daß als proteolytisches Enzym Trypsin, Chymotrypsin, Bromelain oder Papain oder eine Kombination von mehreren dieser Enzyme verwendet wird.

3. Verwendung nach Anspruch 2, **dadurch gekennzeichnet**, daß 20 bis 100 mg Bromelain, 40 bis 120 mg Papain, 10 bis 50 mg Chymotrypsin und 10 bis 50 mg Trypsin pro Dosiseinheit verwendet werden.

4. Verwendung nach einem der Ansprüche 1 bis 3, **dadurch gekennzeichnet**, daß 40 mg Trypsin, 40 mg Chymotrypsin und 100 mg Papain pro Dosiseinheit verwendet werden.

5. Verwendung nach Anspruch 1 bis 4, **dadurch gekennzeichnet**, daß zusätzlich Rutosid verwendet wird.

6. Verwendung nach Anspruch 5, **dadurch gekennzeichnet,** daß 45 mg Bromelain, 60 mg Papain, 1 mg Chymotrypsin, 24 mg Trypsin und 50 mg Rutosid x 3H₂O pro Dosiseinheit verwendet werden.

7. Verwendung nach Anspruch 5, **dadurch gekennzeichnet**, daß 90 mg Bromelain, 48 mg Trypsin und 100 mg Rutosid x 3H₂O pro Dosiseinheit verwendet werden.

8. Verwendung nach einem der Ansprüche 1 bis 7 bei der Behandlung von Erkrankungen und pathologischen Zuständen ausgewählt aus Fibrosen, Arteriosklerosen, Verbrennungen, chronischen Infektionen, insbesondere rheumatischer Arthritis, und Tumoren.

9. Verwendung nach einem der Ansprüche 1 bis 7 zur Vorbeugung der Nebenwirkungen einer Bleomycin-Chemotherapie.

10. Verwendung nach einem der Ansprüche 1 bis 7 bei der Behandlung von Patienten, die einer radioaktiven Bestrahlung unterzogen wurden.

11. Verwendung nach einem der Ansprüche 1 bis 7 bei der Vorbeugung von Abstoßungsreaktionen gegen Implantate oder Transplantate.

12. Verwendung nach einem der Ansprüche 1 bis 7 bei der Vorbeugung oder Behandlung von Diabetes-assoziierten Erkrankungen.

13. Verwendung nach Anspruch 12, **dadurch gekennzeichnet**, daß die Diabetes-assoziierte Erkrankung diabetogene Nephropathie ist.

14. Verfahren zur Beeinflussung von TGF-β, wobei TGF-β mit einem oder mehreren proteolytischen Enzymen in Kontakt gebracht wird.

15. Verfahren nach Anspruch 14, **dadurch gekennzeichnet**, daß zusätzlich Rutosid verwendet wird.
